# EUROPEAN PATENT APPLICATION

(11) **EP 4 640 187 A1**
(43) Date of publication of application: **29.10.2025**
(21) Application number: 24315213.9
(22) Date of filing: 23.04.2024
(51) Int. Cl.: A61F 2/40

(54) **REVERSE SHOULDER ARTHROPLASTY WITH DUAL MOBILITY**

(71) Applicant: Boileau, Pascal, 06300 Nice (FR); Medicalex, 94110 Arcueil (FR)
(72) Inventor: Boileau, Pascal, 06300 NICE (FR); Levy, Fabienne, 75006 PARIS (FR); Levy, Nicole, 75006 PARIS (FR)
(74) Representative: Yes My Patent

(57) **Abstract**

A dual mobility reverse shoulder arthroplasty device for repairing a shoulder articulation,
the device comprising :
- a scapular implant (1), configured to be attached to a scapula of a patient,
- a humeral implant (2), configured to be attached to the humerus of the patient,
- a spherical head (3), rigidly mounted on the scapular implant,
- a mobile head (4), formed as an intermediate cup, mounted on the spherical head with a snap fit assembly manner, the mobile head being able to rotate with regard to the spherical head about an articulation axis, thereby forming a first inner ball joint assembly, the humeral implant comprising an outer cup (21) configured to bear on the mobile head, forming a second outer ball joint assembly, concentric with the first ball joint, thereby achieving large mobility angles of the humeral implant with respect to the scapular implant, and thereby avoiding mechanical impingement at extremal positions.

## Description

### FIELD OF THE DISCLOSURE

The instant disclosure relates to shoulder arthroplasty techniques. Shoulder arthroplasty techniques rely on shoulder prosthetic solutions.

### BACKGROUND OF THE DISCLOSURE

The shoulder is the most mobile and the most unstable joint of the body. Basically, there are two types of shoulder prostheses:
1) Anatomic (hemi or total) shoulder arthroplasty (**ASA**) used when the rotator cuff tendons and muscles are still present and functional, and the bony anatomy of the humerus and scapula is conserved, and
2) Reverse shoulder arthroplasty (**RSA**) used when the rotator cuff tendons are torn and/or the humeral/ glenoid bone stock is compromised, and the anatomy of the proximal humerus or the scapula is distorted. US2021137692 discloses an example of such a reverse shoulder prosthetic solution.

Both types of shoulder prostheses (ASA and RSA) can become unstable with recurrent or chronic dislocations. Revision of unstable shoulder arthroplasty is an incompletely solved problem. While revision surgery using RSA can stabilize the shoulder joint in many cases, there are clinical situations where it is not possible to stabilize it with a RSA despite many reoperations. In case of extensive soft tissue and/or bone deficiency, it may be impossible to resolve recurrent or persistent dislocation of shoulder arthroplasty.

Shoulder prosthesis provides a solution for the treatment of end-stage pathology of the glenohumeral joint in case of bone and/or soft tissue deficiency. However, both *anatomical* and *reverse* shoulder prostheses are potentially unstable. In some cases, prosthetic instability becomes recurrent or chronic, and this complication cannot be solved with current available prostheses.

The instant inventors have endeavoured to further improve the total reverse shoulder arthroplasty solutions, to achieve large mobility angles, to avoid mechanical impingement at extremal positions, to achieve a high stability and to decrease the risks of dislocation.

### SUMMARY OF THE DISCLOSURE

According to one aspect of the present disclosure, it is disclosed a dual mobility reverse shoulder arthroplasty device for repairing a shoulder articulation, the device comprising :
- a scapular implant (1), configured to be attached to a scapula of a patient,
- a humeral implant (2), configured to be attached to a humerus of the patient,
- a first fixed small spherical head (3), rigidly mounted on the scapular implant,
- a second large and mobile head (4), mounted on the small spherical head with a snap fit assembly manner, the mobile head being able to rotate with regard to the spherical head about an articulation axis, thereby forming a first inner ball joint assembly,
the humeral implant comprising an outer cup (21) configured to bear outwardly on the mobile head, forming a second outer ball joint assembly, concentric with the first ball joint, thereby achieving large mobility angles of the humeral implant with respect to the scapular implant, and thereby avoiding mechanical impingement at extremal anatomic positions.

The promoted solution exhibits a higher shoulder stability and decrease the risks of dislocation, while achieving larger mobility angles to avoid mechanical impingements at extremal positions, The design of a "dual head" reverse prosthesis, with one *fixed* small head and one *mobile* larger head, provides increased shoulder mobility and stability.

The snap fit arrangement results in a high retaining force preventing disassembly of the mobile head wit respect to the spherical head.

The reverse shoulder arthroplasty device promoted herein exhibits excellent strength and reliability, the shoulder is well stabilized, the risk of shoulder dislocation is strongly decreased. Thanks to the double ball joint configuration, an enlarged abduction and rotation angle range is provided, as it is discussed further below.

The increased mobility is provided by the double mobility of the large prosthetic head which moves with the small fixed glenoid head on its inner surface and with the humeral cup on its outer surface.

The increased stability is provided by the double mobility of the large head, which delays mechanical contacts between the humerus and the scapula, and by the snap fit fixation system of the large head on the small head which prevents its dislocation

The above solution is named "dual mobility reverse shoulder arthroplasty", e.g. DM-RSA in short. It has been successfully applied to a 52year-old female patient with a largely impaired shoulder area. The implanted solution exhibits an outstanding stability.

Here the wording "shoulder articulation" designate the main shoulder joint linking the humerus head to the glenoid fossa of the scapula; i.e. the glenohumeral joint.

We note here that the term "scapular implant" is considered equivalent to "glenoid implant".

The small spherical head is attached to the distal end of the scapular implant, whereas the proximal end of the scapular implant is fixed to the scapula via fixing means.

In the present disclosure, the term 'to be *attached'* should be construed as encompassing any attachment solution suitable to fix and/or secure an orthopedic implant to a bone member.

It should be noted that the scapular implant can be a single piece part (monobloc) or can be a two-part compound (using a double morse taper for the glenoid neck).

The same is true for the humeral implant that can be a single piece part (monobloc) or a two-part component (using a humeral cup and a stem), or even a three-part component (humeral cup + intermediate neck piece + stem).

We propose here is a dual mobility - reverse shoulder arthroplasty (DM-RSA) designed to be used as a *primary prosthesis* for the treatment of end-stage glenohumeral pathologies and as a *salvage prosthesis* for the treatment of failed shoulder arthroplasty with recurrent or chronic dislocations.

In the known RSA, a large glenoid ball is fixed on the scapula with a baseplate and a large humeral cup is fixed on the humerus; the two components articulate together leading to a Simple Mobility reversed shoulder arthroplasty (**SM-RSA**).

By contrast, in the **DM-RSA** promoted herein, a small glenoid ball is fixed on the scapula with a baseplate and a large humeral cup is also fixed on the humerus; however, there is a mobile head formed as an intermediate cup which is snapped on the small glenoid ball and which is totally mobile; this third piece is mobile inside (with the small glenoid ball) and outside (with the humeral cup) leading to a Double Mobility reversed prosthesis.

The dual mobility - reverse shoulder arthroplasty (**DM-RSA**) is a totally new type of shoulder prosthesis, which use *double ball joint configuration,* designed to solve the problem of shoulder instability without compromising the large mobility of the glenohumeral joint. In cases of failed shoulder arthroplasty (ASA or RSA) with irreducible or chronic dislocation, a DM-RSA can provide the constraints necessary to stabilize the glenohumeral joint and preserve its mobility. The **DM-RSA** is a novel alternative to conventional **ASA** and **RSA** for patients who are at high risk of dislocation and those who present with previous failed and unstable shoulder prosthesis. The goal of the DM-RSA is to prevent postoperative dislocation, while restoring the physiological range of motion of the shoulder and reducing the stresses at the interface.

According to one aspect, the total angular range of the humeral implant with respect to the scapular implant is at least 125°, preferably at least 132°.

Not only the vertical movement is satisfactory, but also the front and rear movement (antero-posterior) exhibits a large angular range, as well as arm rotation. This increases available mobility when compared to single joint prosthetic device.

According to one aspect, the total available angular range of motion comprises a first ball joint angular range comprised between 75° and 90° and a second ball joint angular range comprised between 35° and 55°. Respective angular ranges can be chosen according to patient morphology and material selection.

Advantageously, the mobile head can rotate in all planes regarding the spherical head.

This dual mobility of the mobile head achieves large mobility angles of the humeral implant with respect to the scapular implant, and thereby avoids mechanical impingement or interferences at extremal anatomic positions.

According to one aspect, the mobile head is made of polyethylene. This material has an advantageous sliding surface coefficient. This material is hard enough, but still exhibits a certain elasticity to enable snap fitting. This material is bio-compatible. However, other types of material could potentially be used, such as ceramic, metal or pyrocarbon. A "composite" mobile head, made of two different materials, could also be used. For instance, the mobile head could be made of polyethylene and metal for metal-on-metal articulation (or ceramic-on-ceramic).

It is considered that the first inner ball joint exhibits a smaller resistance to rotation than the resistance to rotation provided by the second outer ball joint. Said otherwise, firstly the mobile head rotates about the spherical head, and when this is not possible any longer, the outer cup rotates about the mobile head.

The large intermediate polyethylene mobile head articulates with the small spherical glenoid head (through its inner surface) and with the large humeral outer cup (through its outer surface).

According to one aspect, the scapular implant is made of titanium. It exhibits an outstanding strength and an excellent ratio strength / weight. Titanium is bio-compatible. This material favours bone adhesion and anchoring. Again, other types of material can potentially be used.

According to one aspect, the humeral implant is made of stainless steel or cobalt-chrome alloy. This material is bio-compatible. This material favours bone anchoring and bone growth in the humeral medulla canal.

According to one aspect, the spherical head is made of stainless steel or ceramic. The skilled person in the art can therefore use known technique to manufacture perfectly spherical outer wall.

According to one aspect, the mobile head exhibits a beveled inner border. This feature favours large angular range and avoid punctual or linear contact point/location between the mobile head and the shaft of the scapular implant.

According to one aspect, the mobile head has an inner wall diameter **D0** comprised in the range [20-30 mm], and preferably comprised in the range [22-24 mm]. This dimension can be chosen according to patient morphology or according to general anthropometrics database.

According to one aspect, the spherical head has a radius R0 comprised in the range [10-14 mm].

According to one aspect, the mobile head has an outer wall diameter **D1** comprised in the range [32-45 mm], and preferably comprised in the range [35-38 mm]. Again here, this dimension can be chosen according to patient morphology or according to general anthropometrics database.

According to one aspect, the scapular implant comprises a glenoid base (10), a shaft (11) centered on a first axis A1, and a spherical head interface. In practice, the shaft is axially interposed between the glenoid base and the spherical head interface. The spherical head interface is typically a 'Morse' tapered cone.

According to one option, the scapular implant may possibly have a central peg (or a central screw) extending along the first axis A1 and configured to be inserted in the scapula, towards the scapula inner area. The central peg (or central screw) provides fixation of the glenoid implant inside the glenoid vault. The fixation of the implant to the scapula is completed with two to four peripherical screws. Secondary fixation of the glenoid implant is obtained through adhesion of bone cells onto the porous metallic surface on the back side. Alternatively, hydroxyapatite can be added on the back surface of the implant to increase bone cells adherence.

According to one option, the glenoid base can have radial and/or lateral protrusion(s) as it will be discussed below.

According to one aspect, the spherical head interface (13) is a Morse cone type.

According to one aspect, the glenoid base has an inclined inner face (16), namely the inner face has a normal with a normal direction angularly spaced from the first axis by an inclination angle (β).

It should be reminded that "a normal to a surface at point P is a vector perpendicular to the tangent plane of the surface".

The inclination angle (β) can be comprised between 15° and 30° and preferably comprised between 20° and 25°. This represents a good compromise to decrease the vertical shear forces undergone by the scapular implant in various use cases and the glenoid fossa can be adapted to receive, via shape complementarity, the inclined face of the glenoid base.

Said otherwise, the top portion of the glenoid base exhibits a greater thickness compared with the lower part of the glenoid base.

On the other side of the glenoid base, the outer face (12) is perpendicular to the first axis.

According to one aspect, the outer cup of the humeral implant exhibits a polished inner surface, configured to slidingly bear on the outer wall or the mobile head.

According to one aspect, there is provided a tapered portion arranged on the shaft, configured to accommodate the extremal positions of the mobile head angular stroke/range.Stated otherwise, there are provided chamfers (18) on the shaft, otherwise named tapered portion. Stated otherwise, the shaft comprises a neck portion. This helps enlarging the angular range of the mobile head. The narrowed portion of the shaft has a diameter D5 comprised between 9 mm and 13 mm, preferably between 10 mm and 12 mm.

According to one aspect, the device may further comprise at least 3 fixing screws to attach the scapular implant to the scapula.

According to one aspect, the glenoid base is provided with at least 3 thru-holes, configured to accommodate said fixing screws.

According to one aspect, the outer cup covers a solid angle of substantially 2π steradian.

According to one aspect, the mobile head (4) covers a solid angle of at least ¾ of a full sphere. Stated otherwise, the mobile head covers a solid angle of at least 3π steradian.

According to one aspect, the mobile head is axisymmetric with regard to a cup axis (A4).

According to one aspect, the scapular implant exhibits prongs/protrusions that can be *linked* to scapula part(s) situated away from the glenoid fossa, e.g. coracoid apophysis, or acromion, or spine of the scapula. This helps further securing the scapular implant in position, taking up vertical shearing forces, thereby preventing dislocation glenoid implant loosening.

Here the term "*Link*" shall be construed broadly, it encompasses 'attach to', or 'bearing on', or any else mechanical interface.

According to one aspect alternatively, the glenoid base with its extensions to the coracoid and scapular spine can be built in one piece or can even be patient-specific and 3D printed.

According to one aspect, the scapular implant is a two-part assembly and comprises a scapular implant socket (8) having an axial bore and a shaft. Thanks to this configuration, the socket can be installed very easily without any hindrance due to the shaft presence, and the shaft is installed thereafter in the axial bore.

### BRIEF DESCRIPTION OF THE DRAWINGS

Other features and advantages of the invention appear from the following detailed description of its embodiments,-given by way of non-limiting example, and with reference to the accompanying drawings, in which:
- Figure 1 illustrates diagrammatically an overview of the promoted prosthetic device, here with the humerus in a lowermost position (max adduction),
- Figure 2 illustrates diagrammatically the promoted prosthetic device, here with the humerus in an uppermost position (max abduction),
- Figure 3 illustrates diagrammatically an exploded view of the promoted prosthetic device,
- Figure 4 illustrates diagrammatically the promoted prosthetic device, with scapula fixing screws,
- Figure 5 shows a perspective view of an example of a mobile head,
- Figure 6 illustrates diagrammatically the angular ranges,
- Figure 7 shows a sectional, transverse view of the glenoid base,
- Figure 8 shows an example of the prosthetic device alone, before use in a patient surgical operation,
- Figure 9 shows an example of an X-ray image of the prosthetic device implanted in a patient shoulder,
- Figure 10 shows a perspective, sectional half-view of an example of a humeral implant
- Figure 11 shows a perspective of a variant embodiment of the scapular implant,
- Figure 12 shows a perspective of another variant embodiment of the scapular implant in a two-part configuration, the part 12A shows the device in an exploded configuration and the part 12B shows the device in an assembled configuration.

### DETAILED DESCRIPTION OF THE DISCLOSURE

In the figures, the same references denote identical or similar elements. For the sake of clarity, various elements may not be represented at scale.

### General overview

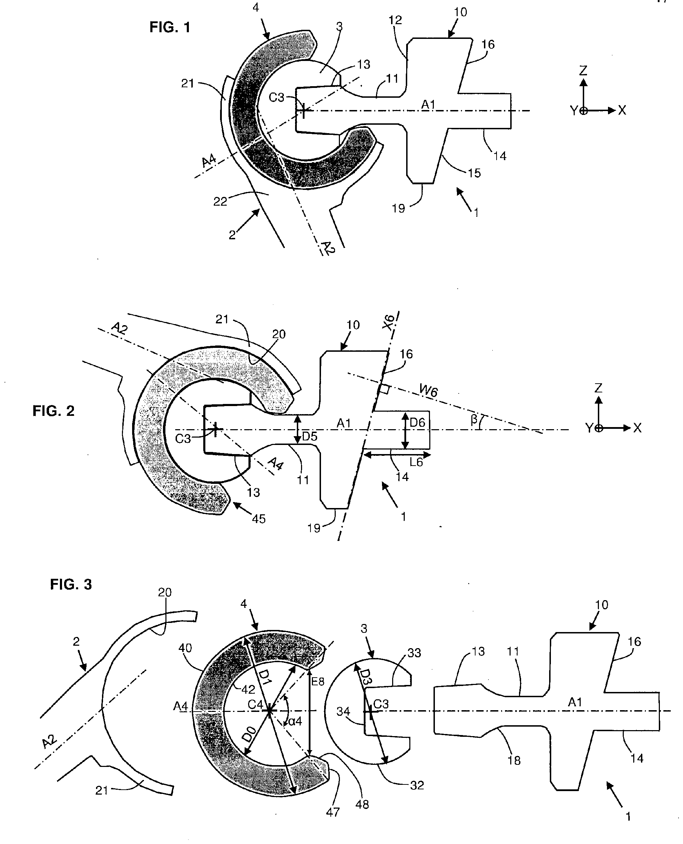

Figure 1 shows an exemplary shoulder prosthetic solution according to the present disclosure. The promoted system is generally a shoulder arthroplasty device designed for repairing a main shoulder articulation, i.e. the articulation between the top portion of the humerus of a patient, and the glenoid seat of the patient's scapula.

The device comprises at least four key components, namely a scapular implant **1**, to be attached to a scapula of a patient, a humeral implant **2**, to be attached to a humerus of the patient, a spherical head **3**, and a mobile head **4**. The structure, arrangement and functions of each component will be discussed in the following sections, in reference to figures 1 to 5. It is to be noted that it is not excluded to have the spherical head and the scapular implant made integrally as a single part. Alternately, the scapular implant can be made in a two-part configuration.

The spherical head 3 and a mobile head **4** can be assembled beforehand, prior to the surgical process, forming a double ball joint sub-assembly.

### Scapular implant - Glenoid implant

In one embodiment, the scapular implant (also named glenoid implant) is made of titanium.

In variant embodiments, the scapular implant **1** can comprise a metallic material such as stainless steel or a metal alloy material such as titanium-based alloy, cobalt-chromium alloy any other biocompatible material.

The scapular implant **1** comprises a glenoid base denoted **10**, a shaft denoted **11**, centered on a first axis **A1**, and a spherical head interface **13.**

The glenoid base can be named metal "metaglene," or glenoid base-plate.

The glenoid base **10** is designed to be coupled or fixed to the glenoid fossa of the patient's scapula.

The shaft **11** is axially interposed between the glenoid base **10** and the spherical head interface **13**, and the shaft 11 has to withstand the shearing and bending forces of all patient use cases.

In one embodiment, the spherical head interface **13** is also centered on the first axis **A1**. In one embodiment, the spherical head interface **13** is a Morse taper cone type.

The scapular implant may possibly have a peg or a central screw (or posterior protrusion in general) extending along the first axis A1 and configured to be inserted in the scapula, towards the scapula inner area. As seen on figure 2, the central peg or screw **14** is a hollow cylinder having an outer diameter **D6** and a length **L6. D6** can be comprised between 8 mm and 10 mm. **L6** can be comprised between 25 mm and 40 mm.

In the illustrated embodiment at figures 1 to 9, the glenoid base **10** is a disk like member centered on the first axis **A1.** The glenoid base **10** is delimited by a front face **12,** also named outer face in accordance with the anatomic intended position, a back face **16** also named inner face in accordance with the anatomic intended position, and a peripheral rim **19.**

The inner face **16** is intended to bear on the glenoid seat **SC,** which has possibly been mechanically prepared beforehand the implant operation. Preparation can involve rubbing and/or reaming.

The inner face **16** has a flat surface along a plane **X6.** The normal direction **W6** is angularly spaced from the first axis **A1** by an inclination angle β. (see figure 2).

The inclination angle β is comprised between 15° and 30° and preferably comprised between 20° and 25°. This represents a good compromise to decrease the shear forces undergone by the scapular implant in various use cases and the glenoid fossa can be adapted/reshaped to receive, via shape complementarity, the inclined face of the glenoid base. The glenoid surface must be reamed to accommodate the glenoid base **10.**

Said otherwise, the top portion of the glenoid base exhibits a greater thickness compared with the lower part of the glenoid base.

Alternatively, the posterior face of glenoid base can have curved surface or be patient - specific and 3D-printed to adapt to the specific glenoid bone loss or erosion.

On the other side of the glenoid base, the outer face **12** is perpendicular to the first axis **A1**.

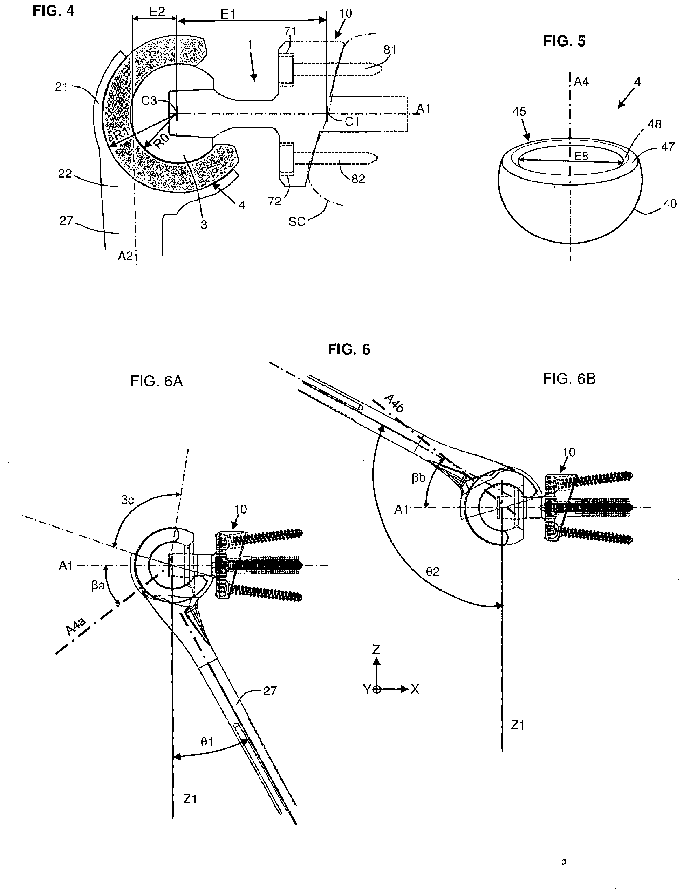

The glenoid center **C1** is located at the center of the inner face **16** as visible at figure 4.

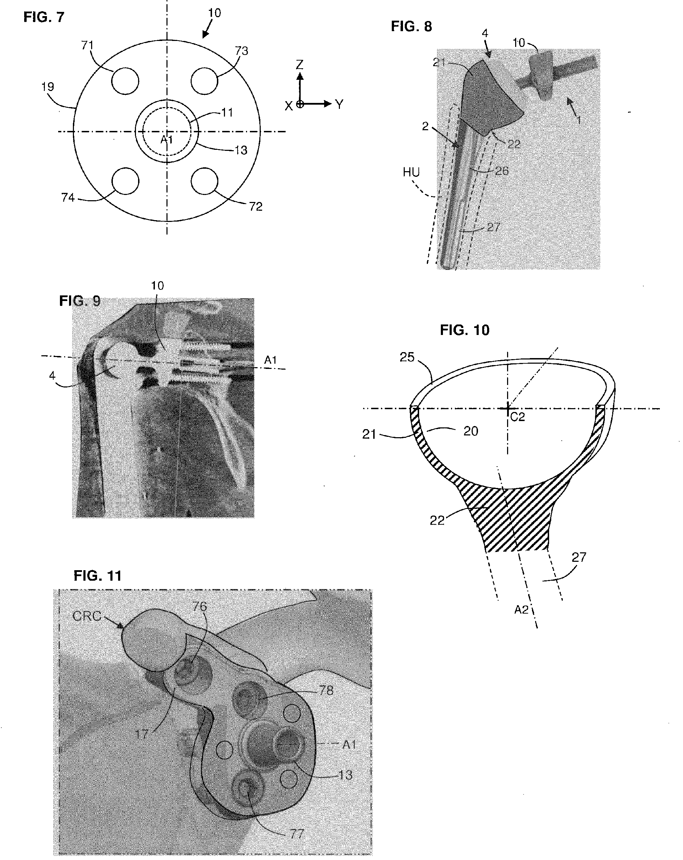

The glenoid base **10** is provided with thru-holes **71,72,73,74** as visible at figure 7, distributed around the axis A1. The thru-holes are configured to receive screws **81**, **82**.

The axis of screws can diverge slightly from the first axis A1, see Figure 6. Alternately, the axis of screws can be slightly convergent.

Variant embodiments of the scapular implant are discussed in the last sections of this disclosure.

### Humeral implant

In one embodiment, the humeral implant **2** is made of stainless steel or cobalt-chrome alloy.

In variant embodiments, the humeral implant **2** can comprise a metallic material such as stainless steel or a metal alloy material such as titanium-based alloy, cobalt-chromium alloy any other biocompatible material.

The humeral implant comprises an implant body **22.** The shape and configuration of the implant body **22** can be designed in accordance with the possibly damaged humerus head or humerus top portion. Therefore, the shape and configuration of the implant body **22** can be highly customized and can therefore exhibit various shapes and arrangements.

The humeral implant comprises an outer cup **21** designed to bear on the mobile head discussed below.

As seen on Figure 4, the outer cup **21** is delimited radially inwards by a hemispheric wall **20.** The center of the cup is denoted **C2.** Once assembled on the mobile head 4, C2 and C3 coincide.

The hemispheric inner wall **20** is polished to result in a very low friction coefficient of the second ball joint.

The outer cup **21** is delimited by a free border **25.** In one embodiment, the free border 25 is a circle centered on cup center **C2,** thereby the cup is exactly a hemisphere.

In variant embodiments, the coverage of the cup might slightly exceed a hemisphere to result in a snap fit assembly on the mobile head **4,** thereby benefiting from the elasticity of the mobile head **4.**

The humeral implant comprises a humerus interface stem denoted **27** extending from the implant body **22.**

As seen on Figure 8, the humerus interface stem **27** is configured to be received in the patient humerus **HU.** The outer surface **26** of the stem has a granularity that can be customized to favour bone adhesion and growth. The stem **27** can be inserted in the medular cavity of the patient humerus **HU.**

Alternatively, the humeral implant cand have a long stem, a short stem or be stemless, or be 3D-printed (to adapt the back side of the implant to the distorted anatomy of the eroded glenoid surface).

In case of tumor or bone loss of the proximal humerus (after fracture sequelae or humeral loosening), a massive metallic, reconstructive humeral prosthesis can be built with the same concept (see radiograph Figure 9 and CT-scan of the treated patient). Alternatively, a humeral implant with an extra-long stem can be implanted inside the distal (native) humerus with use of humeral allograft proximally.

### Spherical head

In one embodiment, the spherical head 3 is made of stainless steel or ceramic.

In variant embodiments, the spherical head can be made of other suitable biocompatible material.

The spherical head has a radius **R0** comprised in the range [10-14 mm]. The diameter **D3** is substantially identical to **D0.** In particular embodiments, for standard cases, **D0** is comprised between 22 mm and 24 mm. However, larger head diameters (>28mm) can be used.

The center of the spherical head **3** is denoted **C3.**

The spherical head is not a full sphere. The spherical head has a conical recess **33,** in accordance to a mechanical interface known as 'Morse' interface.

The shape of the conical recess **33** is a cone with a small diverging angle, delimited axially by a bottom wall **34** (cf Fig 3).

The spherical head **3** is designed to be impacted onto the spherical head interface **13** pertaining to the scapular implant **1** discussed above. The spherical head 3 is in a fixed position, with reference to the scapula.

The outer surface **32** is smooth and polished. A very low friction coefficient is achieved.

### Mobile Head - Intermediate cup

As seen on figure 5, the mobile head **4** is axisymmetric with regard to a cup axis **A4.**

The mobile head **4** has an inner diameter denoted **D0** and an outer diameter denoted **D1.** In some embodiments, **D0** is comprised in the range [20-30 mm]. In some embodiments, **D1** is comprised in the range [32-45 mm]. These dimensions can be chosen according to patient morphology and/or according to general anthropometries database.

The common centre of the inner wall **42** and the outer wall **40** is denoted **C4** (cf Figure 3).

The mobile head **4** is delimited by a generally circular border **45.** In some embodiments, this circular border exhibits an inner bevel **48** and an outer bevel **47.**

As depicted on figure 2, the inner bevel **48** allows to enlarge the angular range of the mobile head **4** around the spherical head.

The outer wall **40** is spherical and matches with the hemispheric inner wall **20** of the outer cup **21.**

The circular border is located at an angle **α4** (Fig 3). Angle **α4** can be comprised between 80° and 120°. Opening has a size **E8** designed to allow a force insertion on the spherical sphere 3.

Said otherwise, the mobile head **4** covers a solid angle of at least ¾ of a full sphere. Stated otherwise, the mobile head covers a solid angle of at least 3π steradian.

We note that, in section view, the angle coverage is 360°- **α4**.

In one embodiment, the mobile head **4** is made of polyethylene. This material has an advantageous sliding surface coefficient. This material is hard enough, but still exhibits a certain elasticity to enable snap fitting. This material is bio-compatible.

In variant embodiments, the mobile head can be made of other suitable material. In some embodiments, the mobile head **4** is generally made from crosslinked polyethylene, highly crosslinked polyethylene, poly ethyl-ethyl ketone, ceramic, metal, or any other biocompatible material.

### Surgical process

The prosthetic device can be implanted through an anterior (deltopectoral) approach or a supero-lateral (transdeltoid) approach.

First, the implants are made available and gathered : scapular implant 1, humeral implant 2, spherical head 3, mobile head 4, as presented above.

According to the first possibility, the spherical head 3 and the mobile head 4 are assembled together as a preliminary step, to form a ball joint sub-assembly.

The surgeon incises patient soft tissues (the subscapularis tendon) and installs tissues retractors to expose the proximal humerus and glenoid surface.

The surgeon prepares the scapula SC, notably the glenoid surface.

The surgeon attaches the scapular implant **1** to the scapula of the patient. In one embodiment, this is made by inserting the central peg (or screw) in a drilled hole and securing the peripherical screws **81**, **82**.

The surgeon osteotomizes the humerus head along the anatomical neck of the patient humerus.

The surgeon implants the humeral implant inside the medullary canal of the humerus **HU.** The humeral implant can be press fitted (without cement) or cemented, depending on the surgeon's preference

The surgeon assembles the outer cup of the humeral implant **2** to the mobile head **4.**

The surgeon possibly re-attach tendons.

### Mobility and stability

Once the assembly is done, the mobile head is able to rotate with regard to the spherical head about an articulation axis **C3,** forming a first inner ball joint assembly.

In addition, the outer cup **21** bears on the mobile head **4,** forming a second outer ball joint assembly, concentric with the first ball joint.

As already mentioned, the second ball joint exhibits a larger resistance to rotation, due to its larger contact area, when compared to resistance to rotation of the first ball joint.

We therefore obtain a dual mobility articulation comprising a double concentric ball joint.

The offset between the glenoid center **C1** and the center **C3** of balljoint is denoted **E1** (see figure 4). The offset between humeral axis **A2** and the center **C3** of ball joint is denoted **E2.**

The glenohumeral joint center is now C3/C4/C2.

Figure 6 shows an exemplary angular range chart.

**A1** denotes the reference axis of the shaft and the morse cone 13. Here A1 is represented horizontally, but according to the patient case, A1 can be oriented slightly downwards going away from the scapula. Figure 6 shows a vertical plane (XZ) corresponding to a front plane of the patient.

Thanks to the above mentioned first ball joint, the mobile head **4** can rotate about **Y,** rotate about **X,** and rotate about **Z.** Figure 6 illustrates the rotation about **Y** axis. **Z1** denotes the downward direction. On the left side of the figure, marked **6A,** the humerus implant stem **27** has an axis **A2** oriented toward the sagittal plane, with an angle denoted **θ1** with respect to the downward vertical **Z1.** The angle **θ1** can be comprised between 10° and 30°.

The mobile head **4** is rotated downwards, and its axisymmetric axis **A4a** is deviated from **A1** by an angle denoted **βa**. The angle **βa** can be comprised between 35° and 45°.

On the right side of the figure, marked **6B,** The mobile head 4 is rotated upwards, and its axisymmetric axis **A4b** is deviated from **A1** by an angle denoted **βb**. In addition, the humerus implant stem **27** has an axis **A2** oriented upwards, with an angle denoted θ2 with respect to the downward vertical **Z1.** The angle **θ2** can be comprised between 110° and 130°.

We note that **βa** = **βb**, by symmetry with regard to A1.

The total available angular range is **θ1** + **θ2.** Here, it exceeds 150°. Even with a less optimized configuration, the skilled person in the art understands that the total available angular range **θ1** + **θ2** can easily be greater than 132°. Generally speaking, the total available angular range **θ1** + **θ2** is greater than 125°.

The second ball joint allow the outer cup **21** to rotate on the mobile head **4** about axis Y (among other rotations about X and Z). Taking into account the extremal positions, the relative rotation of the outer cup 21 with respect to the mobile head has an angular range denoted **βc**. In the illustrated example, the angle **βc** can be comprised between 70° and 90°.

In standard embodiments, **βc** can be comprised between 35° and 55°. In standard embodiments, **βa** + **βb** can be comprised between 75° and 90°.

Figure 9 shows an X-ray image of an exemplary prosthetic device implanted in a patient shoulder. This prosthetic device was applied to a 52year-old female patient with a bone loss of the proximal humerus after a tumor resection. The patient presented with a chronic, permanent dislocation of her shoulder prosthesis. Revision with a DM-RSA allowed to obtain a stable shoulder with sufficient mobility for daily life.

### Variant embodiments

As illustrated on Figure 11, the scapular implant **1** comprises a portion denoted **17** that is linked to the base of the coracoid **CRC.** The scapular implant is secured to the scapula by three screws **76, 77, 78.**

The upward portion 17 is secured in part to the coracoid **CRC.**

Generally speaking, in variant embodiments, the scapular implant may exhibit lateral or radial prongs or protrusions that can be linked to scapula part(s) situated away from the glenoid fossa, e.g. acromion, or coracoid apophysis, or spine of the scapula.

This helps further securing the scapular implant in position, taking up vertical shearing forces, thereby preventing glenoid implant loosening or prothesis dislocation.

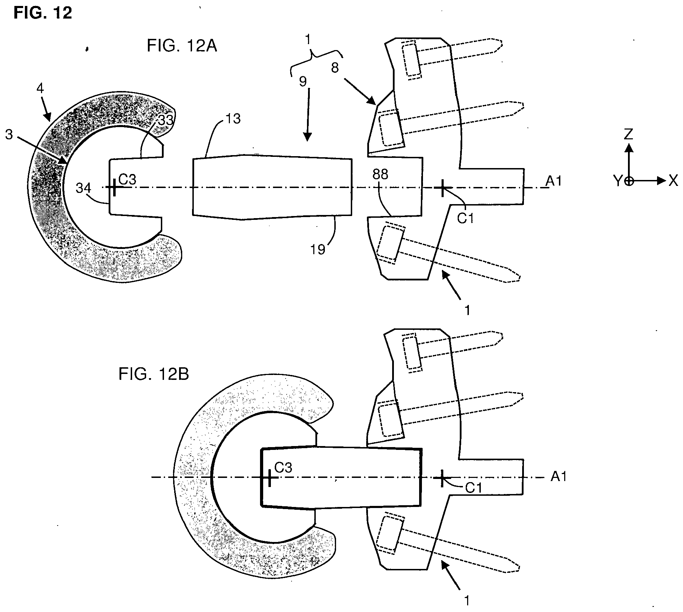

As illustrated on Figure 12 the scapular implant is a two-part assembly (instead of being monolithic). The scapular implant 1 comprises a scapular implant **socket 8** having an axial bore **88.** The scapular implant comprises a shaft **9,** that can be inserted in force into the socket.

The surgeon can place and secure the scapular implant socket **8** in the patient scapula without hindrance from the shaft **9** which is still not assembled at this step stage. Once the scapular implant socket **8** is secured to the glenoid area with the central peg and the peripherical screws, the shaft **9** is inserted into the axial bore inside the socket with a morse taper **88.**

Thereafter the ball joint assembly, comprising the spherical head **3** and the mobile head **4,** is hammered on the Morse cone13 as explained earlier for base variants. The distance **C1** to **C3** can be minimized.

According to a particular embodiment, the device may be provided with a magnetic arrangement between the humeral implant and the spherical head. The arrangement provides an attraction force between the outer cup and the spherical head. This force improves prothesis cohesion and prevents disassembly risk.

## Claims

1. A dual mobility reverse shoulder arthroplasty device for repairing a shoulder articulation, the device comprising:
- a scapular implant (1), configured to be attached to a scapula of a patient,
- a humeral implant (2), configured to be attached to a humerus of the patient,
- a spherical head (3), rigidly mounted on the scapular implant,
- a mobile head (4), mounted on the spherical head with a snap fit assembly manner, the mobile head being able to rotate with regard to the spherical head about an articulation axis (C3), thereby forming a first inner ball joint assembly,
the humeral implant comprising an outer cup (21) configured to bear outwardly on the mobile head, forming a second outer ball joint assembly, concentric with the first ball joint,
thereby achieving large mobility angles of the humeral implant with respect to the scapular implant, and thereby avoiding mechanical impingement at extremal anatomic positions.

2. The arthroplasty device according to claim 1, the total angular range of the humeral implant (2) with respect to the scapular implant is at least 125°, preferably at least 132°.

3. The arthroplasty device according to any of the claims 1 to 2, wherein a total available angular range of motion comprises a first ball joint angular range comprised between 75° and 90° and a second ball joint angular range comprised between 35° and 55°.

4. The arthroplasty device according to claim 1, wherein the mobile head (4) is made of polyethylene.

5. The arthroplasty device according to any of the claims 1 to 2, wherein the scapular implant (1) is made of titanium.

6. The arthroplasty device according to any of the claims 1 to 2, wherein the humeral implant (2) is made of stainless steel or cobalt-chrome alloy.

7. The arthroplasty device, wherein the mobile head (4) exhibits a beveled inner border (48).

8. The arthroplasty device, wherein the mobile head (4) has an inner wall diameter **D0** comprised in the range [20-30 mm].

9. The arthroplasty device, wherein the mobile head (4) has an outer wall diameter **D1** comprised in the range [32-45 mm].

10. The arthroplasty device, wherein the scapular implant comprises a glenoid base (10), a shaft (11) centered on a first axis (A1), and a spherical head interface (13).

11. The arthroplasty device, wherein the glenoid base has an inclined inner face (16), namely the inner face has a normal with a normal direction angularly spaced from the first axis by an inclination angle (β).

12. The arthroplasty device, wherein the outer cup (21) of the humeral implant exhibits a polished inner surface, configured to slidingly bear on the outer wall or the mobile head (4).

13. The arthroplasty device, wherein there is provided a tapered portion (18) on the shaft, configured to accommodate the extremal positions of the mobile head angular stroke/range.

14. The arthroplasty device, further comprising at least 3 fixing screws to attach the scapular implant to the scapula.

15. The arthroplasty device, wherein the outer cup (21) covers a solid angle of substantially 2π steradian.
